# EUROPEAN PATENT APPLICATION

(11) **EP 2 782 055 A1**
(43) Date of publication of application: **24.09.2014**
(21) Application number: 13159812.0
(22) Date of filing: 18.03.2013
(51) Int. Cl.: G06Q 10/06, G06Q 50/22

(54) **Personalised medicine system displaying a timeline of clinical patient information**

(71) Applicant: Optimal Medicine Ltd, London EC3V 3ND (GB)
(72) Inventor: Munro, Janet, 34380 St Martin du Londres (FR); Turner, Rick, Manchester M1 6DE (GB); Tubman, Kenneth L, Millville, MA 01529 (US)
(74) Representative: Miller Sturt Kenyon

(57) **Abstract**

A medical system comprising: a processor; memory; an input module; and an output module, said medical system being configured a patient timeline, said timeline comprising: a date of at least one consultation on a first axis and a heading for each of a plurality of categories of patient data along a second axis; and a plurality of cells, wherein each cell is associated with a category of patient data and at least one consultation. A method and computer program are also provided.

## Description

### Field of the Invention

The present invention relates to a personalised medicine system, and in particular relates to a system that displays a timeline of clinical information about a patient.

### Background of the Invention

Clinical decision support systems are known and such systems can display patient specific clinical information to clinicians, for example for review when considering how to treat a patient. Such information may be displayed in a timeline, charting how a particular characteristic of the patient (for example, blood pressure, weight, blood sugar levels and so on) varies over time. In general, however, such timelines must be viewed in separate displays and do not give the clinician a clear overview of current treatment, symptoms, side effects and other aspects of an illness or other condition or how these characteristics are changing overall and in relation to each other. Accordingly, it can be difficult and/or time consuming for a clinician to form the required overview of the patient and illness or condition needed to decide on an appropriate treatment regimen.

### Summary of the Invention

The present invention seeks to address these shortcomings by providing a personalised medicine system that displays a patient specific clinical timeline to a clinician. According to a first aspect of the present invention, there is provided a medical system comprising: a processor; memory; an input module; and an output module, said medical system being configured a patient timeline, said timeline comprising: a date of at least one consultation on a first axis and a heading for each of a plurality of categories of patient data along a second axis; and a plurality of cells, wherein each cell is associated with a category of patient data and at least one consultation.

Preferably, the system is further configured to display at least one page at the same time as the timeline, each page being associated with a stage in a workflow for guiding a clinical consultation.

Preferably, the system is configured, where patient data has been input during a consultation, to populate the cell corresponding to the date of the consultation and the category of the patient data with an indication that the patient data has been obtained.

Preferably, the indication is the patient data.

Preferably, the system is the indication is a name of a sub-category of patient data, indicating that the obtained patient data is in the sub-category.

Preferably, the indication includes an indication of how the patient data has changed compared to the corresponding patient data obtained for a previous consultation.

Preferably, cells in at least one said category corresponding to adjacent consultations are merged if the patient data is unchanged between consultations.

Preferably, the system is configured, in response to an input indicative of a new consultation, to display a new consultation date on the first axis and to associate a cell in each category of patient data along the second axis with the new consultation date.

Preferably, the system is configured, in response to patient data input by a user on a displayed page during a consultation, to display an indication that the patient data has been obtained in the cell corresponding to the date of the consultation and the category of the patient data.

Preferably, the system is configured to display the timeline as the first page in the workflow.

Preferably, the system is configured to display each page together with at least one line of the timeline.

Preferably, the system is configured, in response to a user selection of a heading, to display a graph of changes in the patient data in the category corresponding to the selected heading.

Preferably, the system is configured, in response to a user selection of a cell, to display a page in the workflow for inputting patient data corresponding to the category of data and the date of the consultation.

Preferably, the system is configured to accept an input of patient data, whereby a user can adjust the patient data by at least one of adding new data and changing existing data, the adjusted patient data being indicated in the timeline.

According to a further aspect of the present invention, there is provided a method, comprising displaying pages, each page being associated with a stage in a workflow for guiding a clinical consultation, receiving user input of patient data during a consultation, storing patient data associated with the consultation, and displaying a patient timeline, said timeline comprising: a date of each consultation on a first axis and a heading for each of a plurality of categories of the patient data along a second axis; and a plurality of cells, wherein each cell is associated with a category of patient data and at least one consultation.

According to a further aspect of the present invention, there is provided a computer program stored on a computer-readable medium for causing a computer to carry out a method comprising: displaying pages, each page being associated with a stage in a workflow for guiding a clinical consultation, receiving user input of patient data during a consultation, storing patient data associated with the consultation, and displaying a patient timeline, said timeline comprising: a date of each consultation on a first axis and a heading for each of a plurality of categories of the patient data along a second axis; and a plurality of cells, wherein each cell is associated with a category of patient data and at least one consultation.

The timeline comprises a matrix of cells each cell corresponding to a particular consultation and a category of patient data and including an indication of what specific data has been recorded and preferably whether and/or how it has changed. In particular, the timeline preferably displays trending data, for example by displaying an arrow next to the indication or by displaying the indication in different colours. The timeline may be displayed alone or at the same time as pages representing different steps in a clinical workflow. It is preferred that the timeline is used to navigate to the relevant pages of the clinical decision support system for more detailed information or to interact with the relevant functionality of the system.

### Brief Description of the Drawings

Embodiments of the present invention will now be described by way of further example only and with reference to the accompanying drawings, in which:
Fig. 1 is a schematic diagram of the architecture of a device for use in the present invention;
Fig. 2 is a schematic diagram of a system according to the present invention;
Fig. 3 is a table representing a workflow for use in the present invention;
Fig. 4 is a partial representation of a screen for display in the present invention, including a header, another display page and a guidelines area;
Fig. 5 is a partial representation of an actions from last visit display page for use in the present invention;
Fig. 6 is a partial representation of an actions due display page for use in the present invention;
Fig. 7 is a partial representation of another display screen for use in the present invention, including two display pages and a guidelines area;
Fig. 8 is a partial representation of another display screen for use in the present invention, including a display page and a guidelines area;
Fig. 9 is a partial representation of another display screen for use in the present invention, including a header, a display page and a guidelines area;
Fig. 10 is a partial representation of another display screen for use in the present invention, including a header, a display page and a guidelines area;
Fig. 11 is a representation of a pop-up display for a symptom rating scale for use in the present invention;
Fig. 12 is a partial representation of a symptom severity sliders display page for use in the present invention;
Fig. 13 is a partial representation of side effect rating scale display page for use in the present invention;
Fig. 14 is a partial representation of another display screen for use in the present invention, including a header, a display page and a guidelines area;
Fig. 15 is a partial representation of a side effect severity sliders display page for use in the present invention;
Fig. 16 is a partial representation of another display screen for use in the present invention, including a header, a display page and a guidelines area;
Fig. 17 is a partial representation of another display screen for use in the present invention, including a header, a display page and a guidelines area;
Fig. 18 is a partial representation of a side effect relative risk display page for use in the present invention;
Fig. 19 is a partial representation of a drug information display page for use in the present invention;
Fig. 20A is a partial representation of a timeline for use in the present invention;
Fig. 20B is a partial representation of a visit selection display for use in the present invention;
Fig. 20C is a partial representation of another timeline for use in the present invention; and
Fig. 21 is a partial representation of a display screen with a minimised timeline for use in the present invention.

### Detailed Description

### General Architecture

The present invention provides a personalised medicine system, which may be a specialised electronic device having only the function of the personalised medicine system. More usually the personalised medicine system of the present invention is an existing electronic device, such as a computer, adapted to have the functionality required by the present invention. Moreover, the personalised medicine system of the present invention may be distributed between several electronic devices/computers which are connected by one or more of a wired LAN, a wireless LAN, a WAN and the Internet.

Where the personalised medicine system is embodied as single electronic device it may be an existing mobile communications device such as a smart phone (for example iPhone^{™} or Android^{™} mobile/cell phone) or a tablet computer (for example iPad^{™} or Samsung Galaxy^{™} tablet). Such a mobile communications device can be adapted to have the functionality of a reminder device according to the present invention by downloading an application or widget to the mobile communications device. In some embodiments, it may interact with other devices to provide the functionality. In others, the mobile communications device may operate in a stand alone fashion.

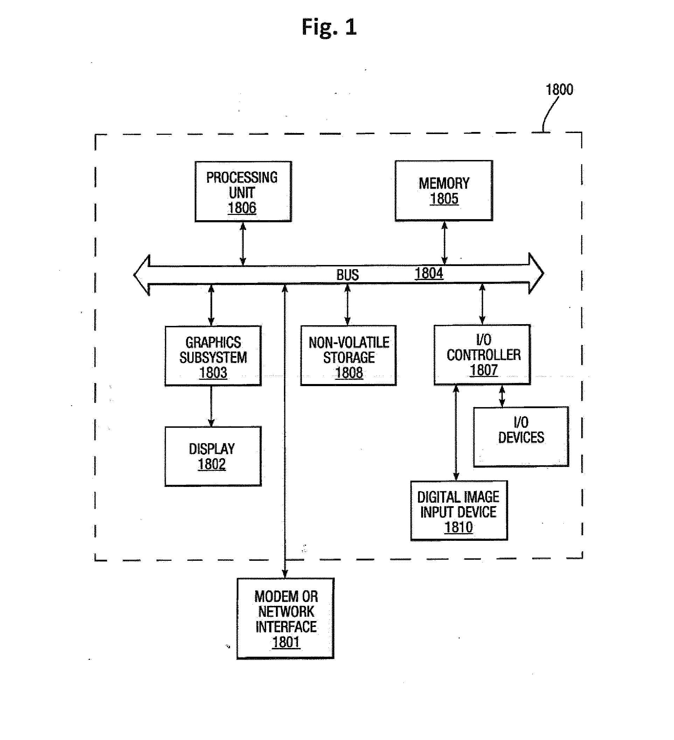

Fig. 1 illustrates an exemplary computer architecture 1800 by which a device embodying or forming part of the personalised medicine system according to the present invention may be implemented. Computer architecture 1800 may be or form part of a desktop computer or a laptop computer, a server, a mobile communications device or any similar computer device.

The computer architecture 1800 may interface to external devices such as another computer, the cloud or Internet, through a modem or network interface 1801, such as an analogue modem, ISDN modem, cable modem, token ring interface, or satellite transmission interface. The network interface 1801 may also be a standard communications module adapted to communicate with a standard mobile communications network, such as 2G, GSM, GPRS, EDGE, 3G, UTMS, CDMA 2000, HDSPA, LTE, 4G, etc networks; a Bluetooth radio; a Wi-Fi radio; or a combination of any two or more of the foregoing.

As shown in Fig. 1, the computer architecture 1800 includes a processing unit 1806, which may be a conventional microprocessor, such as commonly provided by Intel, ARM or AMD, which are known to one of ordinary skill in the computer art. System memory 1805 is coupled to the processing unit 1806 by a system bus 1804. System memory 1805 may be a DRAM, RAM, static RAM (SRAM) or any combination thereof. Bus 1804 couples processing unit 1806 to system memory 1805, to non-volatile storage 1808, to graphics subsystem 1803 and to input/output (I/O) controller 1807. Graphics subsystem 1803 controls a display device 1802, such as a liquid crystal display device, which may be a touchscreen device and/or may be part of the graphics subsystem 1803. The other I/O devices may include one or more of a keyboard, disk drives, printers, a mouse, a speaker, a camera 1810 and the like as known to one of ordinary skill in the computer art.

In one embodiment, the personalised medicine system device 1 is implemented using an application or a widget loaded onto a mobile communications device (such as a smart phone or tablet computer) having a touch screen display device 1802 and a camera 1810 and a speaker (not shown) as I/O devices, and optionally a microphone (not shown) as a further input device. The application causes the device 1 to store predetermined data in the non-volatile storage 1808, which is used to provide the required functionality.

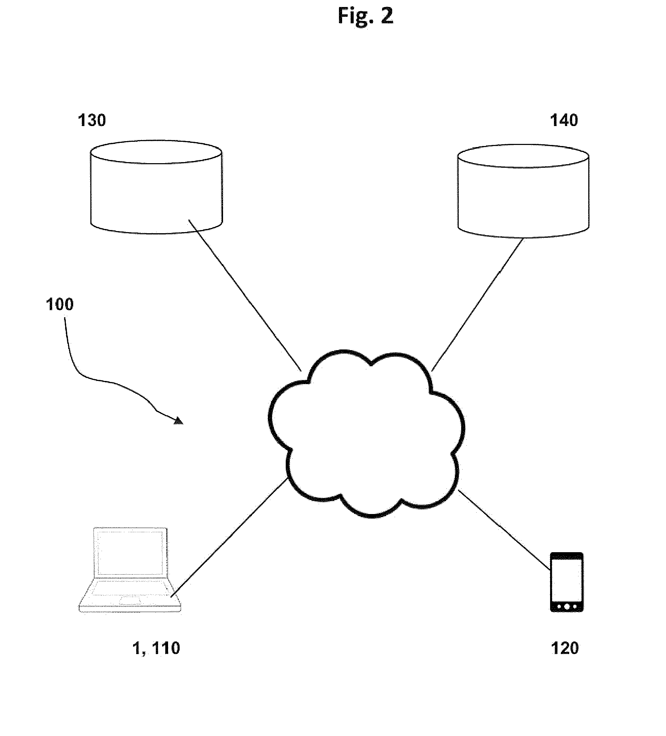

Fig. 2 shows a personalised medicine system 100 comprising a clinician computer 1, 110, a patient smartphone 120, and external servers/computers 130, 140 all of which are connected by one or more of a LAN, a WAN and the Internet. As will be discussed in detail below, the personalised medicine system stores various data, such as workflow information, page information, patient information, slider placement calculation information, side effect severity calculation information, knowledge base information and so forth. Such information may all be stored only on the clinician computer 1, embodying a personalised medicine system of the present invention by itself, or distributed between the clinician computer 110 and one or more networked or remote servers/computers 130, 140, which together embody the personalised medicine system 100 of the present invention. Likewise, processing operations required to provide the functionality of the personalised medicine system 100 may be carried out by the clinician computer 1 alone or may be distributed between the clinician computer 110 and the servers/computers 130, 140. Accordingly, where this specification discusses a particular device such as device 1, 110 storing particular information or carrying out a particular function, it should be understood that in different embodiments the storage and/or functions may be carried out by another device 130, 140 as well or instead.

The patient smartphone, tablet computer or other patient device 120 may provide information to the system 100, such as a record of patient adherence to a treatment regimen, as will be discussed in more detail below.

In some embodiments, multiple clinicians' computers 110 and/or patient devices 120 may be included in the system of the present invention.

### General Operation

The personalised medicine system 1, 100 operates to guide a clinician through a series of interviews with a patient to assist the clinician to gather information about the patient and to decide on appropriate treatment. Thus, the personalised medicine system 1, 100 acts as a clinical decision support system (CDSS) and provides a personalised patient pathway to tailor diagnosis and treatment of an illness or other medical condition to specific patients. In the following description, the personalised medicine system 1, 100 will be described with reference to schizophrenia as an example of an illness/medical condition to which the personalised medicine system is tailored. However, it should be understood that the personalised medicine system 1, 100 can be tailored to other illnesses/medical conditions such as rheumatoid arthritis and cancer, as well as being applied to illnesses/conditions in general.

In order to carry out this functionality, the personalised medicine system 1, 100 stores a plurality of pages for displaying to a user and one or more workflows, each of the pages being associated with a step or node in a workflow. Each node in the workflow has one, two more pages associated with it.

In the present embodiment, the personalised medicine system 1, 100 stores three workflows, Workflow 1 for a first consultation with a patient, where the patient has not previously reported a contact with a psychiatrist or other mental health professional; Workflow 2 for a first consultation where the patient has previously reported a contact with another psychiatrist or mental health professional; and Workflow 3 for a subsequent consultation using the personalised medicine system 1, 100.

When a user (who will generally be a psychiatrist or other clinician) first starts the personalised medicine system 1, 100, for example by clicking an icon on a desktop or tapping an icon on the touch screen display device 1802 of a tablet computer, or navigating their browser to a specific address on a PC or mobile device, the personalised medicine system 1, 100 first displays a welcome screen (not shown). Here the user is able to select an existing patient previously stored in the system 1, 100 or create a new patient. If the user creates a new patient in the system, he is given the option to select whether or not this is patient's first psychiatric contact. Depending on the choices the user makes at this stage, one of the three respective workflows is selected. Each of the three workflows includes a plurality of stages, comprising one or more steps; one or more nodes associated with each step; and one or more pages associated with each node. One or more knowledge bases may be used to populate various pages. Commencement of a workflow starts a "visit", representing a consultation between the clinician and the patient during which information is reviewed and or recorded in the personalised medicine system 1, 100.

As will be discussed in more detail below, users can also access previous visits and use the workflow to move around the visit to review and/or adjust previously recorded information, as well as to add new information.

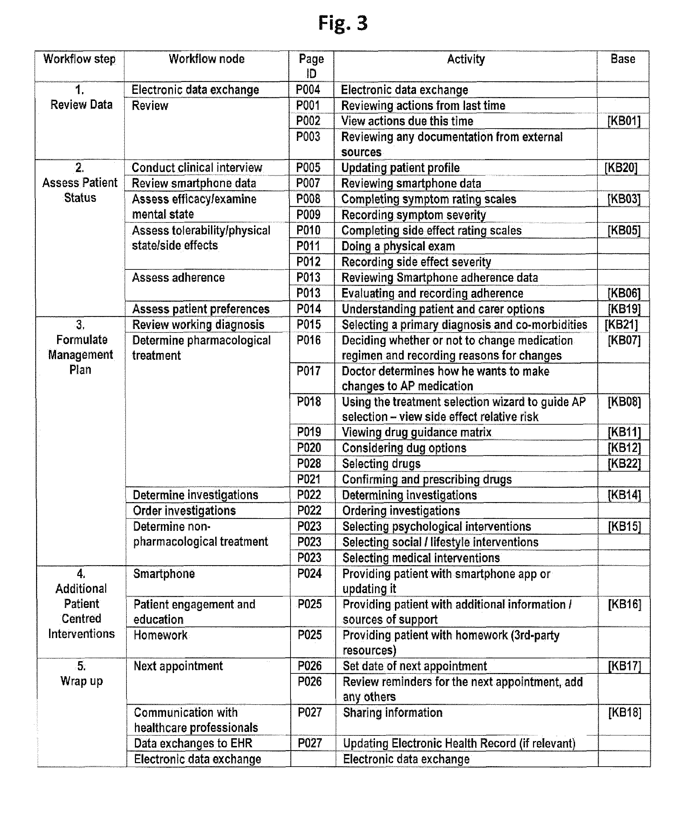

Fig. 3 shows in tabular form Workflow 3 provided for a subsequent consultation by way of example. The other two workflows are similar but changed to take account of their different purposes. The present invention will be described by way of example with reference to the workflow shown in Fig. 3 with relevant changes to the other workflows explained below.

The first workflow step in Fig. 3 for a new consultation with an existing patient is to Review Data and includes two nodes - Electronic Data Exchange and Review. The Electronic Data Exchange node is associated with page ID P004 (not shown). This allows the user to cause the personalised medicine system 1, 100 to interact with other healthcare IT technologies in the clinic/hospital/health authority to carry out automatic data exchange, for example to import patient details stored in electronic health/medical records (EHRs or EMRs) elsewhere, to view previously prescribed and to prescribe drugs, to order lab tests and obtain the results of previously ordered tests. This node may also allow the user to manually enter data, particularly where this is the first psychiatric contact in Workflow 1.

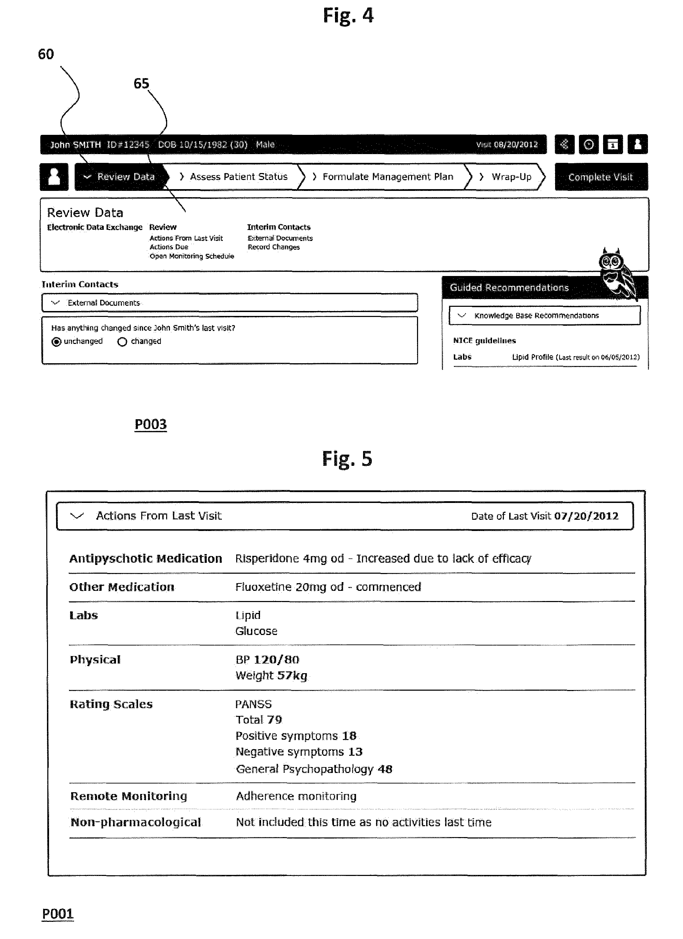

The workflow then moves to the Review node, causing it to display an associated screen. An extract of the screen heading is shown in Fig. 4, which includes the workflow steps in a main heading area 60 and the nodes for the selected step in a sub-heading area 65. The sub-heading area 65 also displays for each node a list of the related activities for which the associated pages are provided. Users can navigate through the workflow by clicking on the respective steps to select them. Once selected, a step is highlighted and a screen sub-heading shows each of the nodes for that step and each of the pages associated with the node. Thus, the user is also able to navigate between nodes and pages by clicking on them to select them. This display structure allows the user to navigate through the workflow in an easy way. Preferably, however, NEXT and PREVIOUS buttons and/or arrows are displayed at the bottom of the screen or elsewhere to allow the user to navigate through a workflow sequentially during a consultation.

In Fig. 4 it can be seen that the user is in the Review Data step of the workflow and is provided with options to enter the Electronic Data Exchange node (discussed above) and the Review node. Within the Review node, the user is able to access pages for reviewing actions from the last visit [page ID P001], viewing actions that are due to be taken this visit [P002] and reviewing documentation from external sources [P003]. In fact, in Fig. 4 the implementation is slightly different from the workflow in Fig. 3 and the option to review documentation from external sources is provided in a separate Interim Contacts node, where it is envisaged that the clinician will view documents created and information changed during an interim contact of the patient with another medical professional since the last consultation. Such interim contacts may occur, for example, because the patient has been admitted to hospital or has obtained psychiatric treatment from another person while on an extended stay at another location. The workflow may be as shown in Fig. 3 or illustrated in Fig. 4.

If the user selects the Review Actions from Last Visit option, for example by clicking on the corresponding wording using a mouse, then page P001 shown in Fig. 5 will be inserted in the screen shown by the display device 1802. Although not shown in Fig. 5, heading area 60 and sub-heading area 65 would also be displayed. Page P001 shows which actions took place as a result of the last visit. Thus, Fig. 5 shows that in the last visit the patient was prescribed risperidone as an antipsychotic medication, fluoxetine as other medication, lipid and glucose labs were ordered, blood pressure and weight were measured, various rating scales were used to assess the patient's symptoms and side effects suffered by the patient, and so forth.

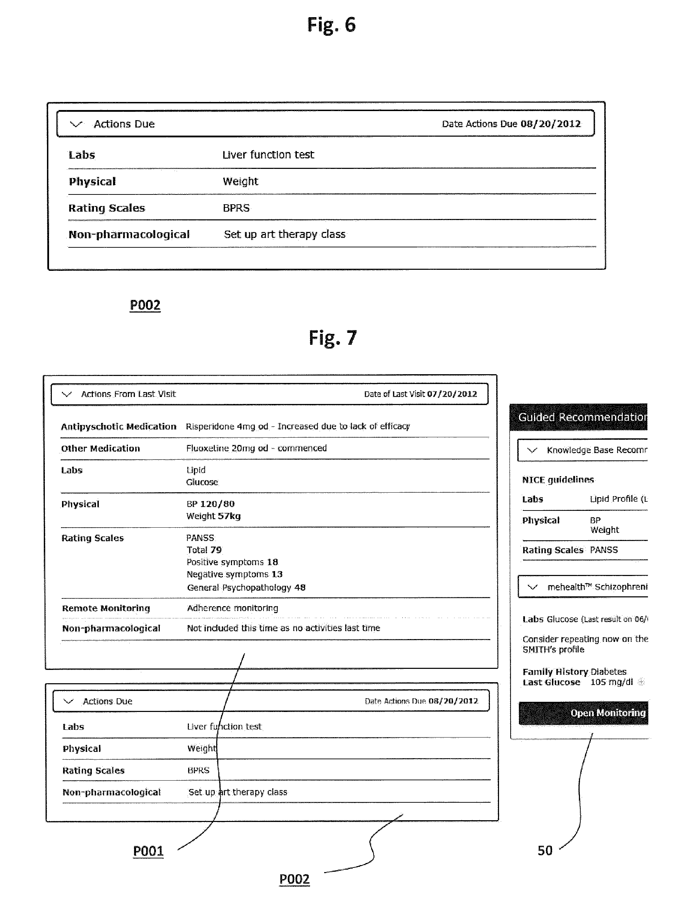

Fig. 6 shows the page P002 by way of further example, which provides the user with a list of actions that should be taken during this consultation.

As illustrated in Fig. 7, in some cases the personalised medicine system 1, 100 may display two or more pages at the same time on the display device 1802. In particular, Fig. 7 shows the concurrent display of pages P001 (actions from last visit) and P002 (actions due). Moreover, as shown in Fig. 7, in addition to displaying the selected pages P001, P002 and P003, the personalised medicine system 1, 100 may simultaneously display guided recommendations for the clinician in guidelines area 50. As before, heading area 60 and sub-heading area 65 would also be displayed simultaneously.

In workflow 3, following completion of the Review Data step, the workflow moves to the Assess Patient Status step and in particular the first node of conducting a clinical interview. In workflows 1 and 2, since this is the first time the patient has been entered in the personalised medicine system 1, 100, there can be no review of existing data and no due actions will have been set. Accordingly, workflows 1 and 2 omit the Review node of the Review Data step and, following electronic data exchange, move directly to the Assess Patient Status step of the workflow.

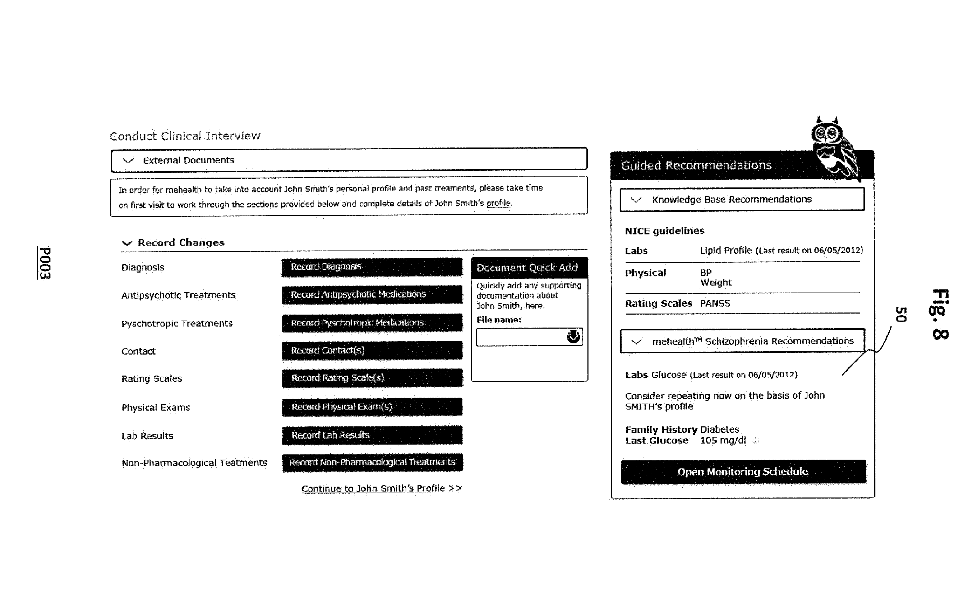

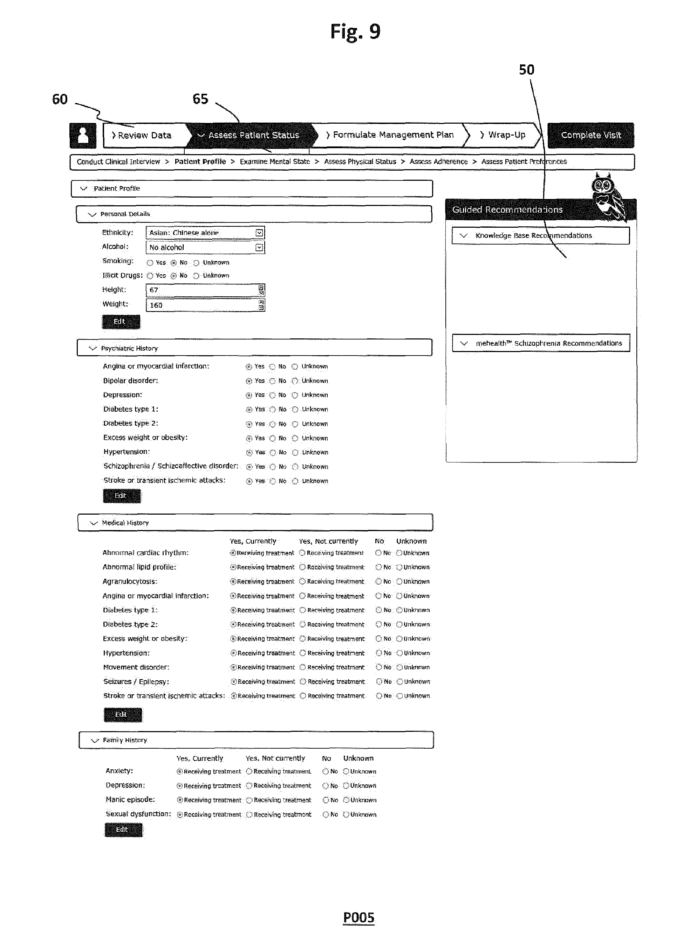

In the Assess Patient Status step of workflows 1 and 2, the first node of conducting a clinical interview is associated with pages P003 (discussed above in respect of workflow 3) and P005. In workflow 3, the conduct clinical interview node is associated only with page P005. Fig. 8 shows an example of page P003 in workflows 1 and 2 together with guidelines area 50. Fig. 9 shows an example of page P005 with guidelines area 50. In workflows 1 and 2, the clinician records the patient profile and clinical history from any available source, including the patient, by systematically working through the prompts in page P003. As shown in Fig. 8, these may include an existing (if any) diagnosis, antipsychotic treatments, other psychotropic treatments, contacts, rating scales, physical examinations, results of laboratory tests, non-pharmacological treatments and so on. Similarly, the clinician completes the patient profile in workflows 1 and 2 or updates the previously-completed patient profile in workflow 3 by following the prompts shown in Fig. 9.

Workflows 1 and 2 then move onto the "examine mental state" node, whereas workflow 3 first allows the clinician to review smartphone data in the "review smartphone" node in conjunction with page P007. In particular, during the (preferably first) visit, the patient's smartphone 120 is updated with an application that allows him to record when he takes his medications and how he is feeling at a given time. The data that has subsequently been input by the patient is then transferred from the smartphone or other patient device 120, for example at predetermined times or during a consultation, onto the clinician's computer 1, 110, and is used to populate page P007 for review by the clinician during the consultation.

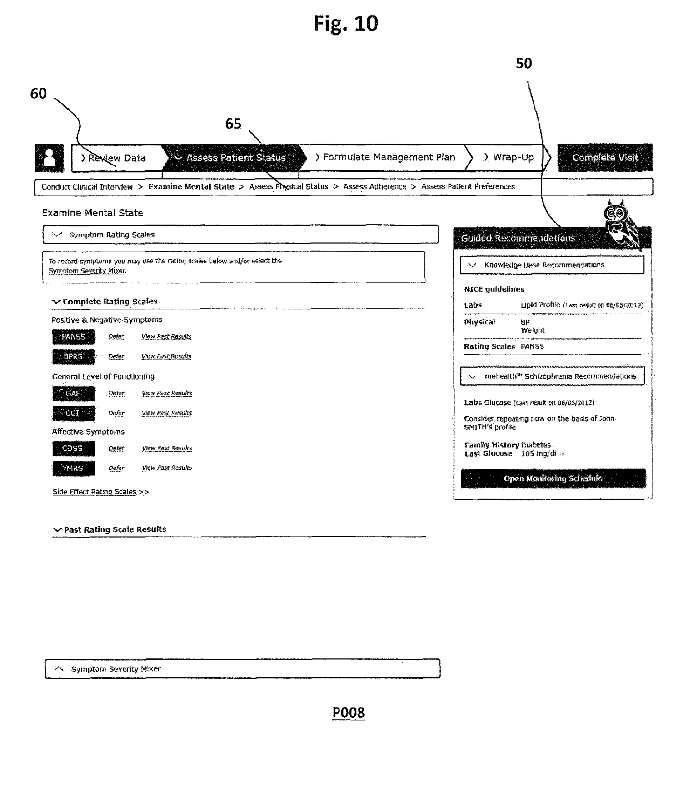

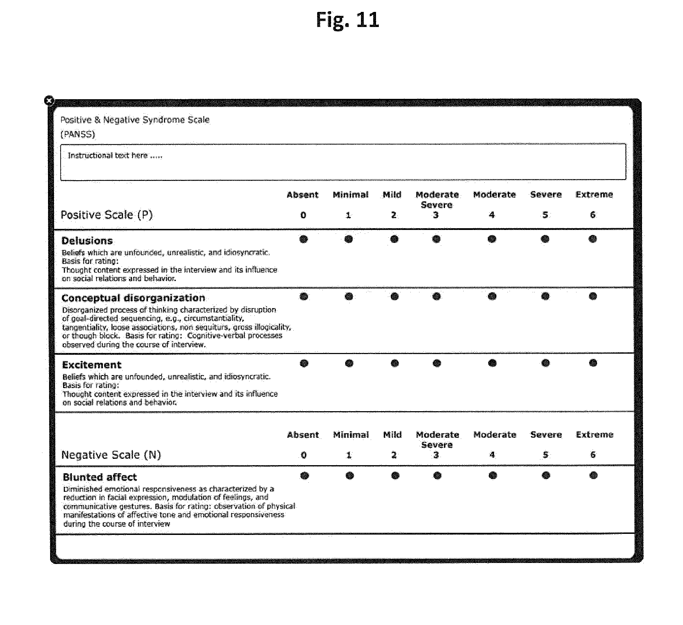

In the "examine mental state" node (which corresponds to the "assess efficacy" node in workflow 3), the user is presented with the option of completing one or more well-established rating scales for assessing the mental state of people suffering psychotic disorders such as schizophrenia. Thus, Fig. 10 shows the display of the screen header 60, 65 highlighting the Assess Patient Status step in the workflow and the "examine mental state" node together with the guidelines area 50. The exemplary symptoms ratings scales shown in Fig. 10 are the positive and negative syndrome scale (PANSS), brief psychiatric rating scale (BPRS), global assessment of functioning (GAF) scale, clinical global impression (CGI) scale, Calgary depression scale for schizophrenia (CDSS) and young mania rating scale (YMRS). However, any number of rating scales may be provided and the user can select any one of the available scales by clicking the corresponding icon to cause a pop-window to open, for example as shown in Fig. 11. The user can then complete the rating scale.

For example, the PANSS is a medical scale used for measuring symptom severity of patients with schizophrenia. To assess a patient using PANSS, an approximately 45-minute clinical interview is conducted. The patient is rated from 1 to 7 on 30 different symptoms based on the interview as well as reports of family members or primary care hospital workers. The symptoms are classified into a positive scale, a negative scale and a general psychopathology scale. The positive sub-scale includes 7 items, minimum score = 7, maximum score = 49 (delusions, conceptual disorganization, hallucinations, hyperactivity, grandiosity, suspiciousness/persecution and hostility); the negative sub-scale includes 7 items, minimum score = 7, maximum score = 49 (blunted affect, emotional withdrawal, poor rapport, passive/apathetic social withdrawal, difficulty in abstract thinking, lack of spontaneity and flow of conversation, and stereotyped thinking) and the general psychopathology sub-scale includes 16 items, minimum score = 16, maximum score = 112 (somatic concern, anxiety, guilt feelings, tension, mannerisms and posturing, depression, motor retardation, uncooperativeness, unusual thought content, disorientation, poor attention, lack of judgment and insight, disturbance of volition, poor impulse control, preoccupation, and active social avoidance).

The other scales operate in a similar manner but include different numbers of symptoms and different scales. For example, the GAF scale is simply a single number selected by the clinician based on his assessment of the patient's overall functioning, based on guidance the scale provides for different number ranges within the scale. Although the different scales may test for different things, generally there is some overlap between scales and different scales may test for (some of) the same things in different ways, for example with different questions and/or different numbers of questions. Other scales may also assess the patient's well-being in different non-clinical domains, for example, quality of life.

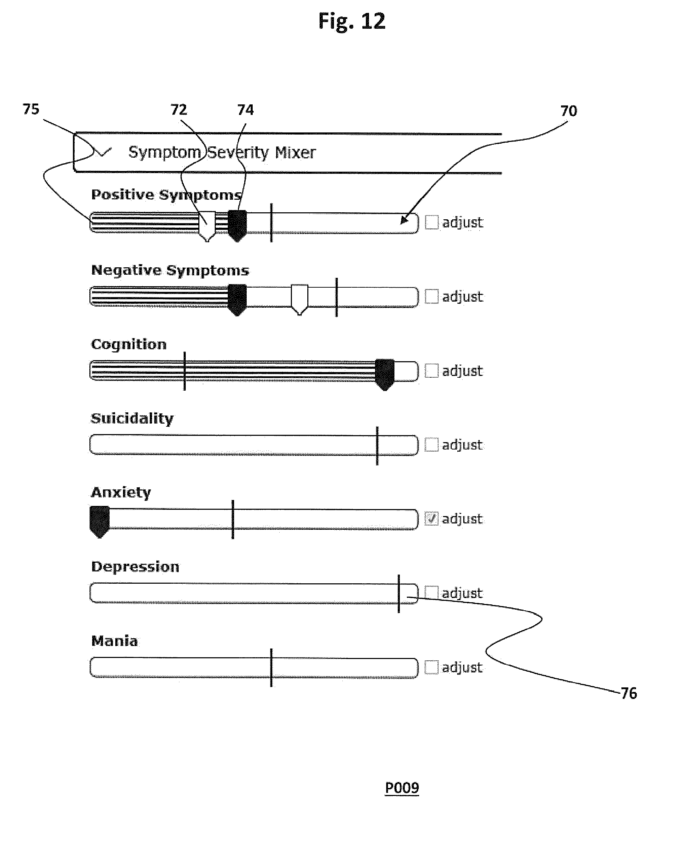

Based on the completion of one or more scales (or none) by the clinician, the personalised medicine system 1, 100 calculates a symptom severity value for each of a plurality of predetermined symptoms and displays the results of the calculations on a graphical scale for each of the symptoms on page P009, as illustrated in Fig. 12. In particular, page P009 shows a graphical representation of linear scale 70 for each of positive symptoms, negative symptoms, cognition, suicidality, anxiety, depression, and mania. Although not illustrated, other scales could also be provided, for example quality of life, and general level of functioning, although this is not an exhaustive list. These graphical representations may be termed symptom (severity) sliders 70 or mixers. In addition to populating the sliders 70 based on the clinician's data input to one or more rating scales in page P008, the user is able to enter a value on the scale based on his own impressions or additional information gained during the visit. Thus, in Fig. 12 shows for each slider 70 a light icon 72 representing a normalised value calculated by the personalised medicine system 1, 100 based on completion of one or more rating scales; a dark icon 74 representing a value enter by the clinician; a fill 75 from 0 up to the dark icon 74 to indicate clearly that the most important value is the clinician updated value; and a line 76 representing the previous value set during the previous visit (which corresponds to the previously set clinician icon 74 or, if the clinician did not input this, the previously calculated normalised value based on completion of one or more rating scales).

Preferably, the user enters a value manually by clicking on the selected slider and dragging an icon along the scale to the appropriate position. If a value has already been calculated by the personalised medicine system 1, 100 or a previous value has been stored, this will be displayed on the slider and the user will be able to use this information together with his experience to position the manually input slider. Alternatively the user may be able to enter a value in box (preferably from 0-10 or 0-100) and this will position the manually input slider on the graphical scale.

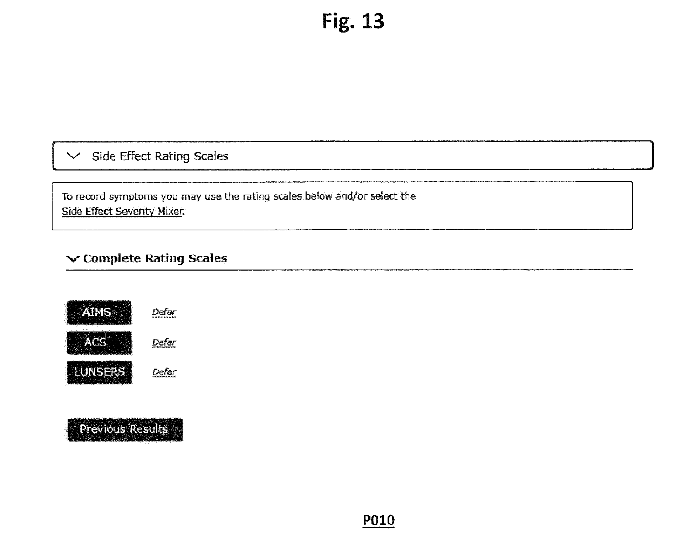

Following completion of the "examine mental state"/"assess efficacy" node, the workflow then moves on to the "assess physical status/side effects"/"assess tolerability" node. This node is associated with page P010, shown in Fig. 13. In this case, the user is given the option to complete one or more predetermined ratings scales to assess side effects of medications experienced by the patient. In the example, the scales are the abnormal involuntary movement scale (AIMS), the antipsychotic side-effect checklist (ASC) and the Liverpool University neuroleptic side effect rating scale (LUNSERS). Again, any number of other rating scales may be provided and again clicking one of the icons causes a pop-up window to open to guide the user through completion of the respective scale.

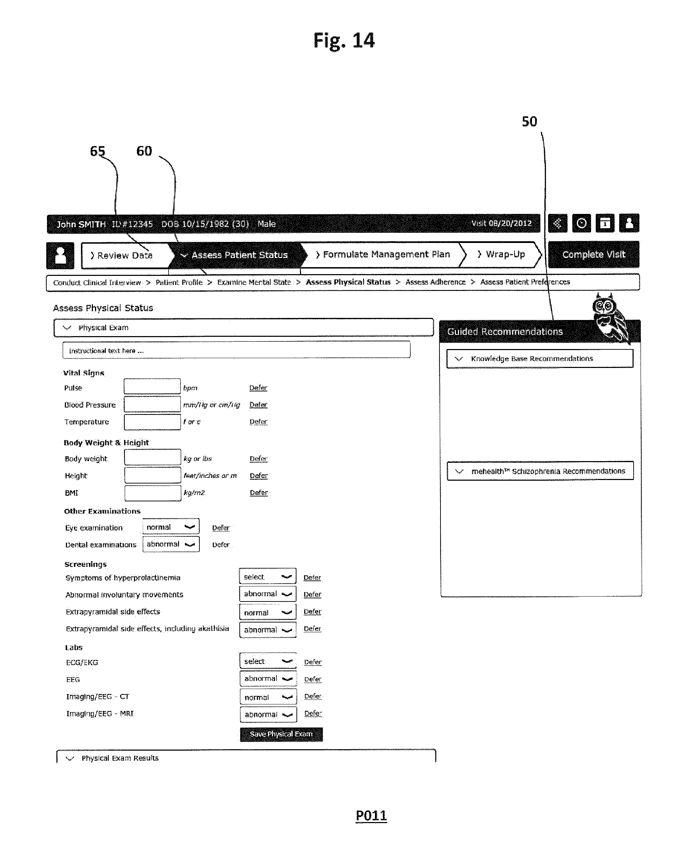

The next page associated with the node is the physical exam page P011. Fig. 14 shows an example of page P011 together with the relevant screen heading 60 and sub-heading 65, as well as the guidelines area 50. As with the other pages, alternative examples are possible. In any event, the user systematically follows the options presented on the page to enter the respective patient details such as height, weight, blood pressure and so forth.

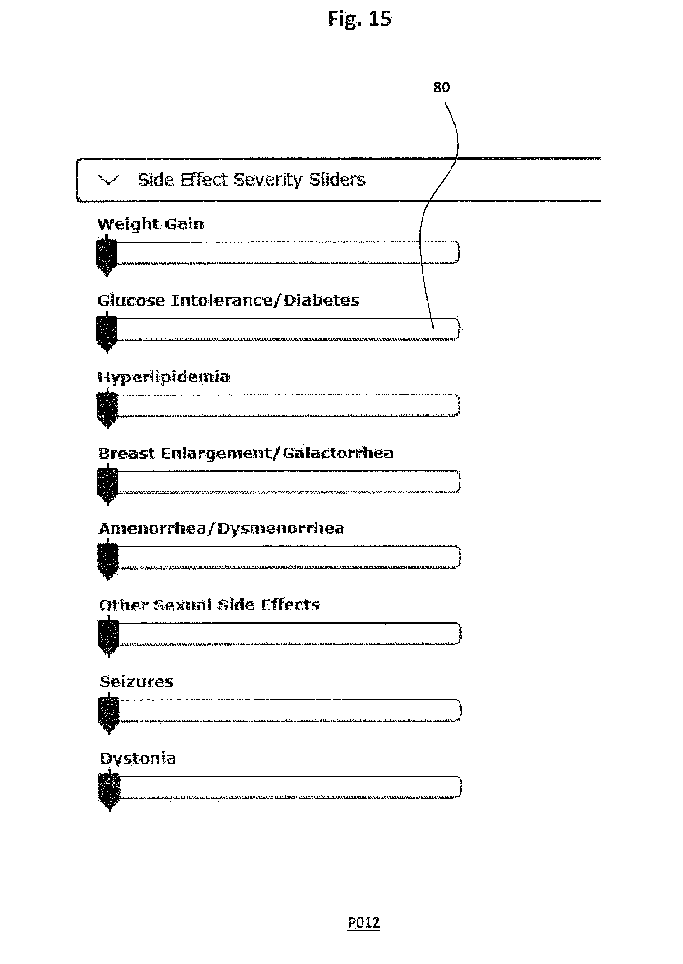

Following completion of pages P010 and P011, the personalised medicine system 1, 100 proceeds to display page P012, shown in Fig. 15, showing side effect (severity) sliders 80. These are populated based upon the completed side effect rating scales and, preferably, the input physical examination data. Similarly to the symptom severity sliders discussed above, the side effect severity sliders include a light icon representing a normalised value calculated by the personalised medicine system 1, 100; a dark icon representing a value manually entered by the clinician; a fill from zero up to the dark icon to indicate clearly that the most important value is the clinician updated value; and a line through the scale representing the previous value set during the previous visit (which corresponds to the previously set clinician icon or, if the clinician did not input this, the previously calculated normalised value based on completion of one or more side effect rating scales).

The user manually enters a value in a side effect severity slider in the same way as discussed above in respect of the symptom severity sliders.

The workflow then moves on to the "assess adherence" node to allow the user to assess how well the patient has adhered to previous or on-going treatment, for example by reviewing data from the patient's smartphone 120 and by checking boxes and entering other information as guided by the corresponding page(s). This node may be omitted or significantly altered in workflows 1 and 2, for example, if the patient is not currently prescribed any medication.

Finally in the Assess Patient Status step, the workflow moves on to the "assess patient preferences" node, where the clinician and patient decide whether to continue with existing medication or to change medication. If a decision to change medication is taken, a pop-up window may open giving the user the option to enter reasons for the change, such as partial efficacy, lack of efficacy or poor tolerability. Again, this node may be omitted or significantly altered in workflows 1 and 2.

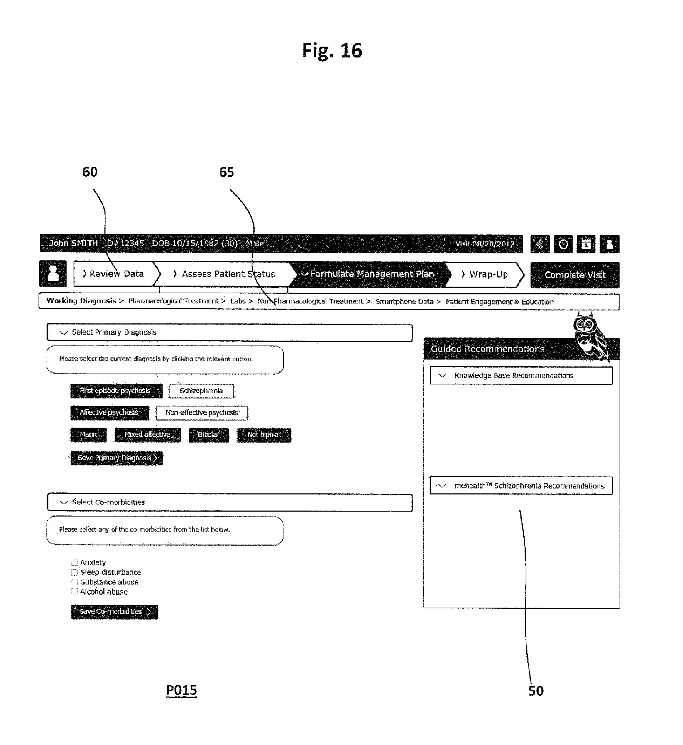

The workflow then moves on to the Formulate Management Plan step. In the first node, "review working diagnosis", page P015 is displayed, as shown in Fig. 16, allowing the user to select or confirm a primary diagnosis and co-morbidities. Preferably, the display of page P015 may change as the primary diagnosis is entered. For example, it may first offer the option of selecting first episode psychosis (FEP) or schizophrenia as the primary diagnosis. If FEP is selected, the page may then offer the selection of affective psychosis or non-affective psychosis. If affective psychosis is selected, the page may then offer the selection of manic, mixed effective, bipolar and non-bipolar. The page also allows the selection of co-morbidities, for example, anxiety, sleep disturbance, drug abuse and alcohol abuse.

In workflows 1 and 2, a "select primary diagnosis" or "determine working diagnosis" node substitute for the "review working diagnosis" node.

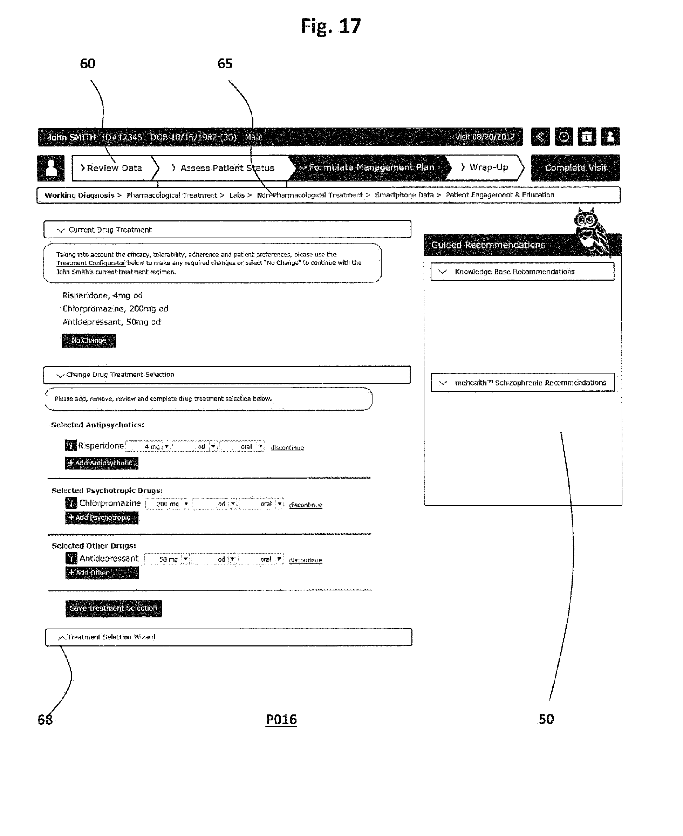

The workflow then moves on to the "determine pharmacological treatment" node, involving the sequential display of pages P016, P017, P018, P019, P020, P028 and P021. Page P016 is shown in Fig. 17 together with heading 60, sub-heading 65 and guidelines area 50. In page P016 the user is shown the current drug treatment regimen and the ability to change the regimen. If the user decides to make a change, pop-up window P017 (not shown) opens requesting the user to select one or more reasons for the decision. As shown in Fig. 17, the user may select one or more new drugs in page P016.

If the user decides not to make any changes, the workflow proceeds to show page P020. In the screen shown on display device 1802, the personalised medicine system 1, 100 provides an option of using an antipsychotic (AP) drug selection wizard or routine provided by the personalised medicine system 1, 100 (see section 68 in Fig. 17) at the same time as showing page P016. If the user decides not to use the wizard, the workflow accepts the changes made in page P016. Otherwise, the workflow moves to page P018.

It will be appreciated that instead of allowing the user to make changes to the drug selection in page P016, an intermediate page may be provided to allow the user either to use the personalised medicine system 1, 100 wizard or to manually select the AP drugs on a new page, which would be similar to the "change drug treatment selection" part shown at the bottom of page P016.

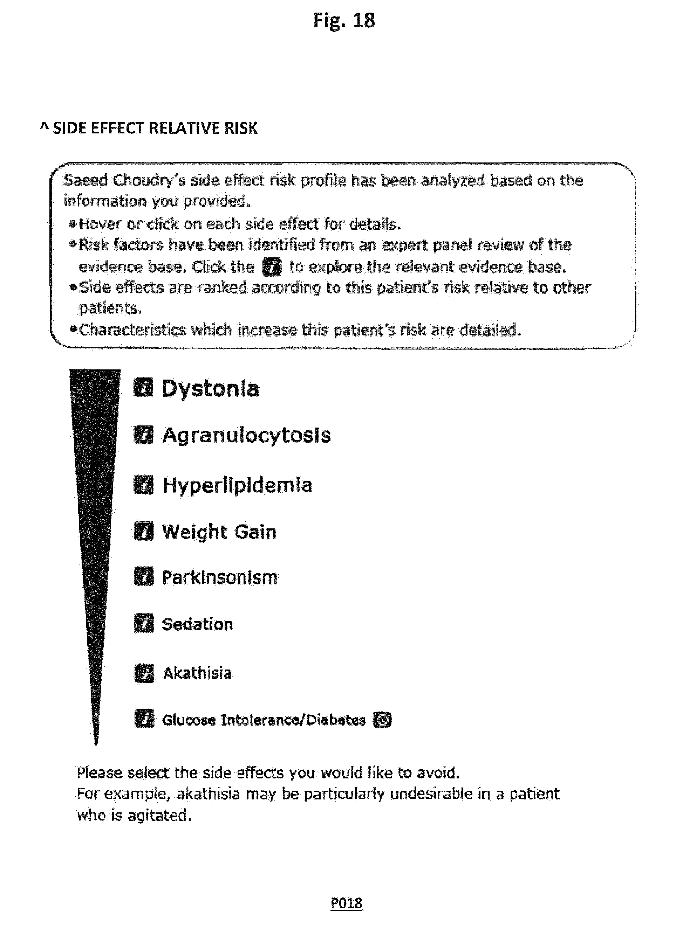

If the user decides to use the wizard, the personalised medicine system 1, 100 may first present, for a plurality of potential side effects, any characteristics which the patient possesses which increase the likelihood of experiencing the side effect. The strength of influence on the side effect of each specific characteristic is presented. The personalised medicine system 1, 100 then calculates, for a plurality of potential side effects, a risk that the patient will experience the side effect relative to the general population. The side effects are then shown in ranked order in page P018, as illustrated in Fig. 18. The manner in which the relative side effect risks and ranked order are determined will be discussed in more detail.

The user may optionally select one or more of the listed side effects which the clinician or patient wants to avoid. For example, a particular patient may particularly want to avoid weight gain or a clinician may recognise that akathisia is undesirable in a patient who is already agitated. A selected side effect may be shown in a different colour, size or font to the other side effects shown on the page.

The user may also hover over or click on each side effect for further details of the side effect, general factors which moderate the side effect, and specific factors extracted from the patient data used in the calculation. The user may also click on an information button next to each side effect to explore the evidence base for the side effect.

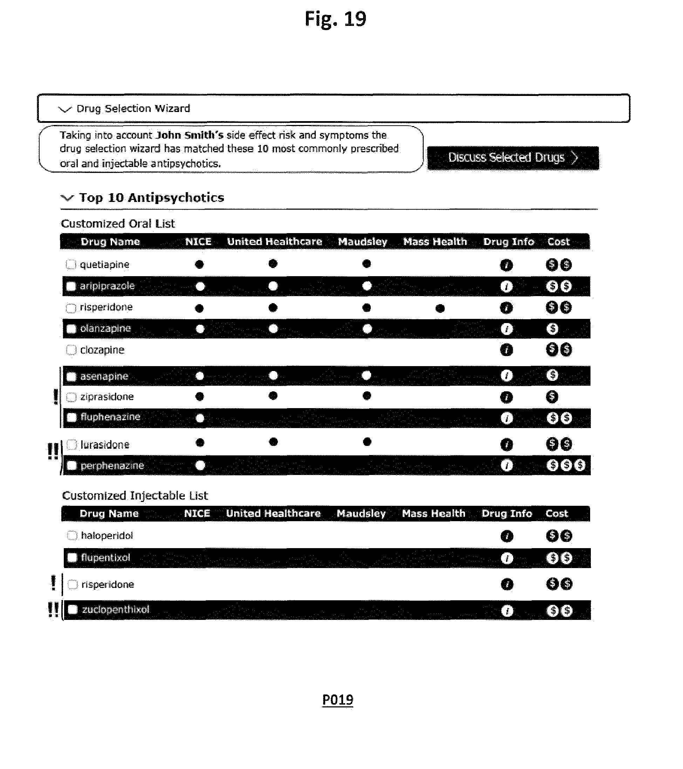

Depending on the working diagnosis selected in page P015, the personalised medicine system 1, 100 then displays in page P019 a list of AP drugs which could be used to treat the patient (see Fig. 19). Moreover, as shown in the figure, the personalised medicine system 1, 100 displays the list of selected AP drugs together with an indication for each drug of whether it is recommended/approved in respective predetermined clinical guidelines by placing a column for each guideline alongside the list of drugs and placing a dot or other marker in each column for each recommended/approved drug. In the example in Fig. 19, the predetermined guidelinesare those published by the UK National Institute for Health and Clinical Excellence (NICE), UnitedHealthcare in the US, the Maudsley psychiatric hospital in the UK, and MassHealth in the US. Naturally, these guidelines are shown by way of illustration only and any other guidelines could be chosen as well or instead.

A column is also provided to allow the user to click on an icon for each drug to obtain further information about the drug, for example from one or more of the guidelines, the manufacturer or other sources. Optionally, the user can click on a dot or other icon under the guideline columns to view information in the respective guideline about the respective drug.

A further cost column is provided showing the relative cost of each drug. Again, clicking on an icon in this column may show the user further information about the costs of the respective drug.

Page P019 further includes an indicator (shown on the left hand side in Fig. 19) of the relative risks of each of the displayed drugs to the particular patient, depending on the side effect relative risks calculated by the personalised medicine system 1, 100, a side effect selected to be avoided in page P018 and, optionally, other patient data. In particular, in the example in Fig. 19, ten drugs to be taken orally are displayed with a single exclamation mark (!) placed against drugs 6-8 in the list and two exclamation marks (!!) placed against drugs 9 and 10. Similarly, five drugs to be taken by injection are displayed with a single exclamation mark (!) placed against drug 4 in the list and two exclamation marks (!!) placed against drug 5. A single exclamation mark may be used, for example, to indicate that the drug should be used with some caution, as the patient's profile suggests that he may have an increased risk of certain adverse side effects. Two exclamation marks may be used to indicate that the drug should be used with extreme caution, as the patient's profile suggests that he is likely to have an increased risk of adverse side effects. Clicking on an icon adjacent the drug may provide further information about what the adverse side effects are and the relative risk of experiencing them. The number of exclamation marks also takes into account the views of the patient and doctor about which side effects would be most undesirable.

Accordingly, the drug table provides a wealth of information tailored to the specific patient to allow the clinician and the patient to discuss suitable AP drug treatment options and select an AP drug for treatment.

Irrespective of whether the pharmacological treatment has been changed, the personalised medicine system 1, 100 then shows page P020 (not shown) which is populated with information about the selected AP drug, allowing further discussion of the treatment options. Subsequently, page P028 (not shown) is displayed, allowing the user to select non-antipsychotic psychotropic medications in addition to the selected AP drug. Optionally, page P028 is only displayed the first time an AP drug has been selected/prescribed or if the AP drug selection has changed. The workflow then moves on to shown page P021 (not shown), in which the user confirms and prescribes the selected drug(s).

The workflow then proceeds to the "determine investigations" node to determine which investigations should be carried out on the patient based on the prescribed drug(s), the patient's status, and other information (such as the evidence base and the guideline recommendations, which are stored in a knowledge base), to obtain baseline values or follow-up evaluations of the patient's tolerability and physical health status; the "ordering investigations" node to order the determined investigations; the "selecting psychological interventions" node for selecting and ordering interventions such as family therapy, cognitive behavioural therapy (CBT) etc; the "selecting social/lifestyle interventions" node for selecting and ordering interventions such as smoking cessation support, gym referral, supported employment etc; and the "selecting medical interventions" node for selecting and ordering interventions such as dietary advice, alcohol/drugs therapy, diabetologist referral etc.

Finally, the workflow moves on to the Additional Patient Centred Interventions and Wrap-up steps of the workflow with their associated nodes and pages.

Accordingly, it will be appreciated that the personalised medicine system 1, 100 of the present embodiment provides a platform written specifically for clinicians and patients in the area of psychiatry, which addresses the unique needs of practitioners as they relate to patient tracking, clinical decision support, clinical references and guidelines, evaluation (treatment and outcome assessment), patient-facing support, and personalised remote self-monitoring. The personalised medicine system 1, 100 provides personalised best practice clinical management decisions by combining industry-recognised guidelines, a published evidence base, a clear workflow for the clinician to follow and the patient's unique characteristics. The present invention improves the care of individuals with chronic mental illness, where there is a large number of different treatments available, each with unpredictable efficacy and troublesome side effects. In addition to providing guidance on the selection of appropriate drugs for treatment of the patient, the personalised medicine system 1, 100 of the present invention also helps the doctor with making non-drug treatment decisions; obtaining, providing and reviewing patient information; monitoring, recording and presenting outcome etc.

Although the present embodiment relates to a personalised medicine system 1, 100 specifically adapted to the treatment of mental illness and, specifically, schizophrenia and other psychosis, it will be apparent that the personalised medicine system 1, 100 of the present invention can be adapted to a host of other illnesses, including especially those illnesses and conditions that require long term care and multiple consultations between patient and physician, such as various different forms of cancer, rheumatoid arthritis and so on. However, the personalised medicine system 1, 100 of the present invention can also be adapted to provide a workflow for the general practitioner or trained nurse to follow in more general consultations in less specialised fields of practice.

In each case, the personalised medicine system 1, 100 of the present invention can provide point-of-care decision support for a wide spectrum of clinical decisions from diagnosis to chronic disease management. It accompanies the doctor and patient across the full continuum of care and throughout the illness, informing therapeutic decisions, monitoring outcomes and offering patient-focused interventions. In doing so, the personalised medicine system 1, 100 allows clinicians to apply the evidence-base to each individual patient and facilitates concordance of the treatment with selected guidelines, promoting consistently higher quality care. Preferably, it can be customised by healthcare providers and doctors to preferred guidelines or formularies.

### Timeline

A further innovative aspect of the present invention is the provision of a timeline. In more detail, a timeline 400 is displayed before commencing the workflow and during the display of the pages as the workflow proceeds.

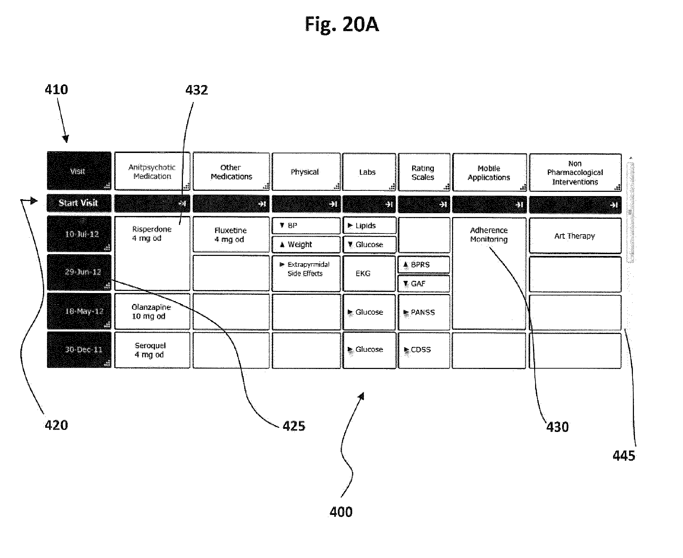

An exemplary timeline 400 is shown in Fig. 20A. This timeline is shown when the user first logs on to the personalised medicine system 1, 100 and selects a patient for consultation. The timeline comprises a header row 410 along a horizontal axis, together with visit rows 420, 425 arranged below the header row 410 along a vertical axis and extending along the horizontal axis.

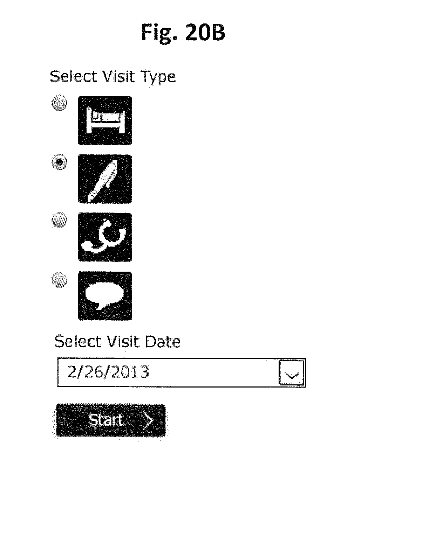

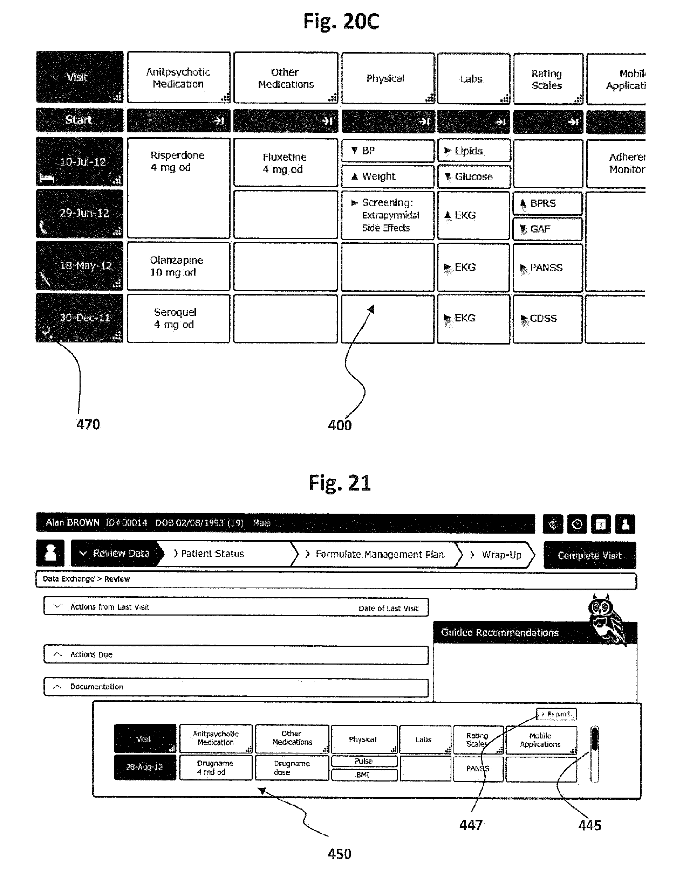

The header row 410 includes headings for each of a plurality of different categories of patient data. In the example in Fig. 20, these categories are antipsychotic medication; other medications; physical (height, weight, blood pressure etc); labs (lipids test, glucose test etc); rating scales (BPRS, PANSS etc); mobile applications (installed on the patient's smartphone or tablet for remote monitoring); and non-pharmacological interventions (CBT, gym, art etc).

Each of the visit rows 420, 425 shows the patient data recorded for the patient for a particular visit. Thus, in the first column of a visit row 425 the date of the consultation is displayed. In subsequent columns of each visit row, a cell is provided. Thus, a cell is provided at the intersection of each heading and each visit, and an indication of the patient data for that visit in the respective category is displayed in each cell.

Accordingly, taking the example shown in Fig. 20, it can immediately be ascertained that the patient visited the clinic on 30 December 2011, 18 May 2012, 29 June 2012, and 10 July 2012. In the visit of 30 December 2011, a CDSS rating scale assessment was performed, a glucose test was ordered and quetiapine was prescribed at 4 mg once per day. The arrows 440 indicate that the results of the glucose test and the CDSS were unchanged (or not significantly changed) from previous results.

During the visit of 18 May 2012, a PANSS assessment was performed, another glucose test was ordered and the antipsychotic prescription was changed to olanzapine at 10 mg once per day. In addition, adherence monitoring was commenced using the patient's smartphone.

During the visit of 29 June 2012, BPRS and GAF assessments were performed, an ECG (EKG) test was ordered, and an assessment of extrapyramidal side effects was carried out. In addition, the antipsychotic prescription was changed again to risperidone at 4 mg once per day. In addition, as indicated by the merged cell 430, adherence monitoring was continued.

Finally, during the visit of 10 July 2012, lipids and glucose labs were ordered and blood pressure and weight were measured. Since last being measured, lipids were constant and glucose levels were reduced, weight having increased and blood pressure having fallen. The antipsychotic prescription risperidone at 4 mg once per day was continued, as indicated by the merged cell 432. In addition, the non-AP medication fluoxetine was prescribed at 4 mg once per day. Adherence monitoring was continued and art therapy was commenced.

Accordingly, at the beginning of a consultation the clinician can quickly assimilate the treatment history of the patient, with details being shown in such a way that changes/continuations in symptoms/treatments/other factors can be seen at a glance. Preferably, data gathered before use of the personalised medicine system 1, 100 and subsequently entered is clearly separated from other data in the timeline, for example by using a different colour, shading or font, or a combination of these.

Advantageously, the timeline is interactive. If the user clicks on one of the headings in the heading row 410, the personalised medicine system 1, 100 is caused to display a series of graphs in a pop-up charting window, illustrating graphically how the different factors recorded in the respective category have changed over time. For example, if the user clicks on the Physical heading, he will be shown graphs of changes in each of the patient's measured physical characteristics, such as pulse rate, blood pressure, temperature, weight, BMI, and so on.

Alternatively, if the user clicks on a cell in the timeline, he is taken to the page in the workflow where the information was entered. For example, clicking on Pulse will take the user to the physical exam page P011 for the visit in which Pulse is located. Effectively then, the personalised medicine system 1, 100 is able to recreate previous workflows and allows the user to navigate through them as with a current workflow. The user may be enabled to edit previous visits, for example to correct previously input information or to add new information. The personalised medicine system 1, 100 informs the user when they are editing a previous visit. Preferably, all input fields within a completed visit are initially disabled, but the user is given the ability to edit a page via a banner at the top of a page that appears when the user first attempts to edit the page.

If the user mouses over a cell in the timeline, a pop-up window may be displayed to see further details of the patient characteristics in the cell - for example, the patient's weight at that visit. As previously discussed, some cells within the timeline are merged. In more detail, cells under headings such as "Antipsychotic Medication", "Other Medication" and "Mobile Applications" are merged across visits where there is continuity between the visits. However, separate cells are started where there has been a change.

Where there have been a large number of visits, the timeline is provided with a scroll bar 445 so the user can scroll between earlier and later visits.

It is preferred that the personalised medicine system 1, 100 allows a distinction to be made between the different times data may be added to the system. To differentiate between the types of data in the personalised medicine system 1, 100 in a preferred embodiment, four visit types are provided: new visit (of the patient to the clinic); admission (an event where a patient is admitted into a clinical setting, for example as an inpatient at a hospital); encounter (an encounter with the patient outside of a scheduled visit, for example by phone or e-mail; and data entry (any data collected between visits to be included as part of the patient record, such as the results of lab tests). Naturally, these are not limiting.

The first row 420 in the timeline (under the heading row 410) is the Start row. If the user clicks on the Start cell (shown as Start Visit in Fig. 20A and Start in Fig. 20C), the personalised medicine system 1, 100 displays the information shown in Fig. 20B and gives the user the option of selecting the appropriate visit type. If the user inputs that the visit is a new visit by the patient to the clinic, the current date will populate the start cell. Otherwise, the user can choose the date to associate with the admission-, encounter- or data entry-type visit. An icon 470 is placed in the visit identifier in the first column, as shown in Fig. 20C, so that the user can distinguish between different types of visit when viewing the timeline 400 later. The personalised medical system 1, 100 this will begin the appropriate workflow and start the display of the respective pages as the workflow progresses.

As shown in Fig. 21, in a preferred embodiment a minimised timeline 450 is permanently shown at the bottom of the display device 1802 as the user progresses through the workflow. The minimised timeline includes the header row 410 and, in the first instance, the current visit row 420 created by clicking in the start cell. If the user has entered the workflow by clicking on a cell from a previous visit, the row corresponding to the previous visit will initially be displayed in the minimised timeline. (The header row in the embodiment shown in Fig. 21 is different from that shown in Fig. 20 simply because two different embodiments of the personalised medicine system 1, 100 are shown. However, if the figures were to relate to the same embodiment, the same headers would be shown in the header row 410 of the two figures.)

As the user progresses through the workflow and inputs patient data (whether in the current visit or a previous visit), this is immediately reflected in the timeline. In other words, the timeline is populated directly patient information is entered/changed. Again, the user can click on cells in the minimized timeline to navigate to the relevant page in the workflow to enter the relevant patient information.

Accordingly, in addition to being able to navigate through the workflow sequentially as described above by clicking forward/next and back/previous buttons or by clicking on the workflow steps and nodes in the display header 60 and sub-header 65, the full timeline 400 and the minimised timeline 450 provide the user with a further way of navigating through the workflow.

As shown in Fig. 21, the minimised timeline 450 is provided with a scroll bar 445 so he can scroll through previous visits even as he is going through the current workflow. The minimised timeline 450 is also provided with a maximise button 447 to allow the user to view the full timeline 450 overlaid on the page display. When the timeline has been maximised in this way, the button 447 operates as a minimise button to allow the user to return to the minimised timeline 450.

As such, the timeline is a powerful tool giving the user quick access to a summary of a patient's progress since initial interaction with the personalised medicine system 1, 100, and even beforehand if the relevant data is correctly input. The timeline is available from all major screens in the personalised medicine system 1, 100. This allows the user to quickly scan through a summary of past visits as well as the data already entered during the current visit even as the user is entering more detailed information using the various pages in the workflow. Moreover, the user can see trends in the data from the timeline due to the arrow indicators 440 and quickly gain access to more detailed information by mousing over a populated cell or by drilling down to the detailed information by clicking on the cell or a heading in the header row 410. As such, the timeline provides many unique advantages in fully informing a clinician's decisions as a consultation progresses.

In addition to, or instead of, displaying the arrows 440, the indication of patient data may be displayed in different colours to show either changes in patient data or trends in patient data. For example, if the particular patient data has deteriorated (for example, blood pressure has increased), then the corresponding indication (for example, the wording "Blood pressure") may be displayed in red and if the particular patient data has improved, the corresponding indication may be displayed in green. Alternatively, another colour such as blue may be used to indicate a change. In addition or instead, the cells may be displayed in different colours to indicate changes or trends.

The foregoing description has been given by way of example only and it will be appreciated by a person skilled in the art that modifications can be made without departing from the scope of the present invention.

For example, the foregoing description describes operation of the personalised medicine system 1, 100 by reference to application to the treatment of mental illness. However, the present invention is not limited to this and can be applied across the spectrum of medical complaints. Although applicable to general practice and any illness or condition, the present invention has particular benefit in the treatment of longer term illnesses and conditions, which require on-going treatment and many consultations.

Although the present invention has been described by reference to consultations and visits by the patient to the clinician, it should be understood that consultations need not be face to face (although this is preferred). Moreover, as discussed above, the personalised medicine system 1, 100 may make provision for "dummy" visits/consultations, for example involving contact of the patient with other medical professionals or for adding the results of lab tests and other investigations.

Where the words "step" and "stage" have been used in this specification, they should not be construed narrowly and, except where the context requires otherwise, they may be considered to be synonymous. Thus, the terms incorporate nodes and sub-nodes as described above. Thus, each page could be considered as being associated with a separate, individual stage or step in the workflow, with these stages or steps being grouped into nodes and higher level stages or steps.

## Claims

1. A medical system comprising:
a processor;
memory;
an input module; and
an output module,
said medical system being configured to display a patient specific clinical timeline, said timeline comprising:
a date of at least one consultation on a first axis and a heading for each of a plurality of categories of patient data along a second axis; and
a plurality of cells,
wherein each cell is associated with a category of patient data and at least one consultation.

2. A medical system according to claim 1, further configured to display at least one page at the same time as the timeline, each page being associated with a stage in a workflow for guiding a clinical consultation.

3. A medical system according to claim 1 or claim 2, configured, where patient data has been input during a consultation, to populate the cell corresponding to the date of the consultation and the category of the patient data with an indication that the patient data has been obtained.

4. A medical system according to claim 3, wherein the indication is the patient data.

5. A medical system according to claim 3 or claim 4, wherein the indication is a name of a sub-category of patient data, indicating that the obtained patient data is in the sub-category.

6. A medical system according to any one claims 3 to 5, wherein the indication includes an indication of how the patient data has changed compared to the corresponding patient data obtained for a previous consultation.

7. A medical system according to any one of claims 3 to 6, wherein cells in at least one said category corresponding to adjacent consultations are merged if the patient data is unchanged between consultations.

8. A medical system according to any one of the preceding claims, configured, in response to an input indicative of a new consultation, to display a new consultation date on the first axis and to associate a cell in each category of patient data along the second axis with the new consultation date.

9. A medical system according to any one of the preceding claims, configured, in response to patient data input by a user on a displayed page during a consultation, to display an indication that the patient data has been obtained in the cell corresponding to the date of the consultation and the category of the patient data.

10. A medical system according to any one of the preceding claims, configured to display each page together with at least one line of the timeline.

11. A medical system according to any one of the preceding claims, configured, in response to a user selection of a heading, to display a graph of changes in the patient data in the category corresponding to the selected heading.

12. A medical system according to any one of the preceding claims, configured, in response to a user selection of a cell, to display a page in the workflow for inputting patient data corresponding to the category of data and the date of the consultation.

13. A medical system according to claim 13, configured to accept an input of patient data, whereby a user can adjust the patient data by at least one of adding new data and changing existing data, the adjusted patient data being indicated in the timeline.

14. A method, comprising
displaying pages, each page being associated with a stage in a workflow for guiding a clinical consultation,
receiving user input of patient data during a consultation,
storing patient data associated with the consultation, and
displaying a patient timeline, said timeline comprising:
a date of each consultation on a first axis and a heading for each of a plurality of categories of the patient data along a second axis; and
a plurality of cells,
wherein each cell is associated with a category of patient data and at least one consultation.

15. A computer program stored on a computer-readable medium for causing a computer to carry out a method comprising:
displaying pages, each page being associated with a stage in a workflow for guiding a clinical consultation,
receiving user input of patient data during a consultation,
storing patient data associated with the consultation, and
displaying a patient timeline, said timeline comprising:
a date of each consultation on a first axis and a heading for each of a plurality of categories of the patient data along a second axis; and
a plurality of cells,
wherein each cell is associated with a category of patient data and at least one consultation.
